(19) **Europäisches Patentamt**
**European Patent Office**
**Office européen des brevets**

(11) Veröffentlichungsnummer: **0 203 513 B1**

(12) **EUROPÄISCHE PATENTSCHRIFT**

(45) Veröffentlichungstag der Patentschrift: **03.07.91**

(51) Int. Cl.⁵: **A61M 1/36**

(21) Anmeldenummer: **86106901.1**

(22) Anmeldetag: **21.05.86**

(54) **Medizinische Beutelanordnung.**

(30) Priorität: **23.05.85 DE 8515209 U**

(43) Veröffentlichungstag der Anmeldung:
**03.12.86 Patentblatt 86/49**

(45) Bekanntmachung des Hinweises auf die
Patenterteilung:
**03.07.91 Patentblatt 91/27**

(84) Benannte Vertragsstaaten:
**DE FR GB IT**

(56) Entgegenhaltungen:
**EP-A- 0 090 093**
**DE-U- 8 106 491**
**DE-U- 8 515 209**
**FR-A- 2 566 273**

(73) Patentinhaber: **Fresenius AG**
**Gluckensteinweg 5**
**W-6380 Bad Homburg v.d.H.(DE)**

(72) Erfinder: **Magasi, Josef**
**Wendelinusstrasse 8**
**W-6902 Sandhausen(DE)**

(74) Vertreter: **Dr. Fuchs, Dr. Luderschmidt**
**Dipl.-Phys. Seids, Dr. Mehler Patentanwälte**
**Abraham-Lincoln-Strasse 7**
**W-6200 Wiesbaden(DE)**

**Beschreibung**

Die Erfindung betrifft eine medizinische Beutelanordnung aus einem organischen Polymerisat zur Perfusion eines Gerätes zur Behandlung des menschlichen Körpers gemäß dem Oberbegriff des Anspruchs 1.

Bei zahlreichen akuten und chronischen Erkrankungen ist es erforderlich, eine Behandlung von Körperflüssigkeiten außerhalb des Körpers im extrakorporalen Kreislauf durchzuführen. Derartige Behandlungen werden bei der Hämodialyse oder Hämofiltration durchgeführt, wobei das Blut in einem Dialysator der Dialysebehandlung unterzogen wird.

Derartige Dialysatoren weisen üblicherweise eine Vielzahl von Membranen auf, die den Dialysator in eine von Blut durchflossene Kammer und eine von Dialysierflüssigkeit durchflossene Kammer trennen.

Derartige Kapillar- oder Plattendialysatoren werden bereits werksseitig sterilisiert, beispielsweise durch die Behandlung mit Ethylenoxid oder durch Bestrahlung mit sterilisierender Strahlung, und mit Hilfe eines Konservierungstoffes, beispielsweise Glycerin, konserviert. Bei Bedarf werden sie dann bettseitig eingesetzt, wobei es erforderlich ist, derartige Blutbehandlungsgeräte vor der Benutzung zu spülen, um die anhaftenden Chemikalien, insbesondere die Konservierungsstoffe, zu entfernen.

Hierzu wird die Blutseite des Dialysators mit einem Blutschlauchsystem verbunden, das eine arteriöse und eine venöse Seite aufweist, deren Enden jeweils ein Konnektorstück zur Aufnahme der Nadeln aufweist. Eine Seite des Blutschlauchsystems wird dabei in eine peristaltische Pumpe eingelegt und es werden die beiden Enden des Blutschlauchsystems mit einem Beutel verbunden, der eine Spülflüssigkeit, beispielsweise eine Kochsalzlösung, aufweist. Anschließend wird die Kochsalzlösung durch den Dialysator gepumpt, wobei zugleich der Dialysator mit der physiologischen Kochsalzlösung gefüllt und von den anhaftenden Chemikalien freigespült wird.

Nachteilig an dieser Anordnung ist jedoch, daß sich die mit den Chemikalien versetzte Spülflüssigkeit bei der Rückführung in den Beutel mit frischer Spülflüssigkeit vermischt, so daß die aus dem Dialysator zu entfernenden Stoffe erneut wiederum in den Spülkreislauf eindringen können.

Es wurde daher eine Beutelanordnung vorgeschlagen, bei der der die frische Spülflüssigkeit enthaltende Beutel von dem die verbrauchte Spülflüssigkeit enthaltenden Beutel getrennt ist. Dies hat den Vorteil, daß die frische Spülflüssigkeit nicht mehr mit der verbrauchten Spülflüssigkeit in Berührung kommt, jedoch den Nachteil, daß der Spülvorgang selbst vom Bedienungspersonal überwacht werden muß, da ansonsten die Gefahr besteht, daß der Beutel mit verbrauchter Spülflüssigkeit platzt und die gesamte Anordnung leerläuft, was neben der unerwünschten Füllung des Dialysators mit Luft auch Sterilitätsprobleme nach sich

Aus der DE-U-8 106 491 ist eine medizinische Beutelanordnung zur Perfusion eines Gerätes zur Behandlung des menschlichen Körpers bekannt, die zwei Beutelkammern aufweist. Die erste Beutelkammer ist hierbei mit Spülflüssigkeit gefüllt und weist eine Auslaßöffnung auf, während die zweite Beutelkammer eine Einlaßöffnung aufweist. Ferner ist eine Verbindungsleitung vorgesehen, die eine Strömungsverbindung zwischen der Einlaßleitung zur zweiten Beutelkammer und der ersten Beutelkammer schafft. Hierbei kann im Betrieb der bekannten Beutelanordnung in die Verbindungsleitung ein Absperrorgan eingesetzt werden.

Die bekannte Beutelanordnung weist jedoch auch wie andere bekannte Beutelanordnungen den Nachteil auf, daß eine Überwachung des Spülvorganges durch Bedienungspersonal erforderlich ist, da dieses beim Auslösen des Alarms die Einlaßleitung schließen und die Verbindungsleitung öffnen muß, da ansonsten der Spülvorgang aufgrund des Stoppens der Pumpe zum Stillstand kommen würde. Daraus resultiert zum einen ein relativ hoher gerätetechnischer Aufwand, da entsprechende Überwachungs- und Anzeige- bzw. Warnvorrichtungen vorgesehen werden müssen, und darüber hinaus ist der Betrieb der bekannten Beutelanordnung zeit- und personalaufwendig.

Es ist daher Aufgabe der vorliegenden Erfindung, eine Beutelanordnung der im Oberbegriff des Anspruchs 1 angegebenen Art zu schaffen, die ein Spülen eines Gerätes zur Behandlung des menschlichen Körpers, insbesondere eines Dialysators, ohne Überwachungsmaßnahmen und bei geringem gerätetechnischen Aufwand ermöglicht

Die Lösung dieser Aufgabe erfolgt durch die Merkmale des kennzeichnenden Teils des Hauptanspruches.

Die erfindungsgemäße Beutelanordnung weist den Vorteil auf, daß sie nach dem Anschluß an den Dialysator und der Inbetriebnahme der peristaltischen Pumpe nicht mehr vom Bedienungspersonal überwacht werden muß. Die Pumpe entnimmt aus der ersten Beutelkammer frische Spülflüssigkeit und führt sie auf der Blutseite des Dialysators an der Membranwand entlang und laugt dabei die in der Membran befindlichen Stoffe, beispielsweise Glycerin oder Natriumacetat, aus der Membran zu Beginn der Spülbehandlung heraus. Die mit diesen Stoffen beaufschlagte Spülflüssigkeit verläßt den Dialysator und kehrt zur Beutelanordnung in die zweite Beutelkammer zurück, wobei diese mit verbrauchter Spülflüssigkeit gefüllt wird. Die Füllung der zweiten Beutelkammer kann nur solange erfolgen, bis das gesamte zur Verfügung stehende Innenvolumen verbraucht ist. Anschließend steigt der Innendruck im Beutel schlagartig an, was üblicherweise zum Platzen des Beutels bzw. zum Stillstand des Spülschritts führen würde. Da jedoch erfindungsgemäß im Bereich der Einlaßleitung zur zweiten Beutelkammer ein Überdruckventil vorgesehen ist, kann die Spülflüssigkeit durch das Überdruckventil entweichen.

Gemäß einer besonders vorteilhaften Ausführungsform der Erfindung ist die Einlaßleitung erfindungsgemäß mit einer Überströmleitung verbunden, die in der ersten Beutelkammer mündet. Dabei ist das Überdruckventil in der Überströmleitung vorgesehen, so daß nach der vollständigen Füllung der zweiten Beutelkammer mit verbrauchter Spülflüssigkeit im wesentlichen unkontaminierte Spülflüssigkeit durch die Einlaßleitung und die Überströmleitung in die erste Beutelkammer strömt, wobei der Strömungskreislauf geschlossen wird, da aus der ersten Kammer praktisch unverbrauchte Spülflüssigkeit zugleich abgezogen wird.

Es hat sich dabei gezeigt, daß die in die zweiten Beutelkammer zupumpende Spülflüssigkeitsmenge ausreicht, um den Dialysator wirksam von den Konservierungssubstanzen zu befreien. Demzufolge wird nach der Füllung der zweiten Beutelkammer über die Überströmleitung praktisch reine Spülflüssigkeit abgeleitet. Anzumerken ist, daß das gesamte System gegenüber der Umgebung geschlossen ist, d.h. der sterile Zustand beim Spülen aufrechterhalten bleibt.

Als Spülflüssigkeit wird üblicherweise physiologische Kochsalzlösung, 0,9%ige NaCl-Lösung oder 5%ige Levulose-Lösung eingesetzt, die auch zur Befüllung des Blutschlauchsystems und der blutseitigen Kammer des Dialysators benutzt wird.

Dialysefilter werden von den Herstellern trocken entweder mit Ethylenoxid oder -Strahlen sterilisiert oder aber mit destilliertem Wasser gefüllt und autoklaviert. Von Seiten der Hersteller wird vorgeschlagen, daß die ersten 300 - 500 ml Spülflüssigkeit bei der Spülbehandlung dieser Filter verworfen werden, da hierdurch gewährleistet wird, daß das in der Membran enthaltene Glycerin, das zur Konservierung der Membran dient, sowie restliche im Filter enthaltene Partikel aus dem System ausgespült werden. Demzufolge hat man nach Entfernung der 300 - 500 ml den venösen Teil des Blutschlauchsystems mit dem die Spülflüssigkeit enthaltenen Beutel gekoppelt, wodurch der vorstehend erwähnte Rezirkulationsvorgang in Gang gesetzt wurde. Daß hierdurch Sterilitätsprobleme entstanden, bedarf keiner weiteren Erläuterung.

Mit der erfindungsgemäßen Beutelanordnung erübrigt sich dieser Arbeits- und Zeitaufwand, da der Perfusionsvorgang selbsttätig abläuft, so daß das Bedienungspersonal lediglich die beiden Enden des Blutschlauchsystems mit der erfindungsgemäßen Beutelanordnung verbinden muß. Der Rest des Spülvorgangs läuft selbsttätig ohne Überwachung ab.

Vorteilhafterweise sind die Enden der Einlaßleitung und der Auslaßleitung jeweils mit einem Anschlußstück versehen, das komplementär mit den an den Enden des Blutschlauchsystems vorgesehenen Anschlußstücken zusammenpaßt. Nach der Perfusionsbehandlung werden die Verbindungen wieder gelöst, wobei die Enden der Blutschlauchleitungen jeweils mit einer Nadel versehen werden.

Weitere Vorteile, Einzelheiten und Merkmale der Erfindung werden anhand der nachfolgenden Beschreibung von zwei Ausführungsbeispielen unter Bezugnahme auf die Zeichnung erläutert.

Es zeigen:

Fig. 1    schematisch eine erste Ausführungsform der Erfindung im Querschnitt und

Fig. 2    eine zweite Ausführungsform der erfindungsgemäßen Beutelanordnung im Querschnitt.

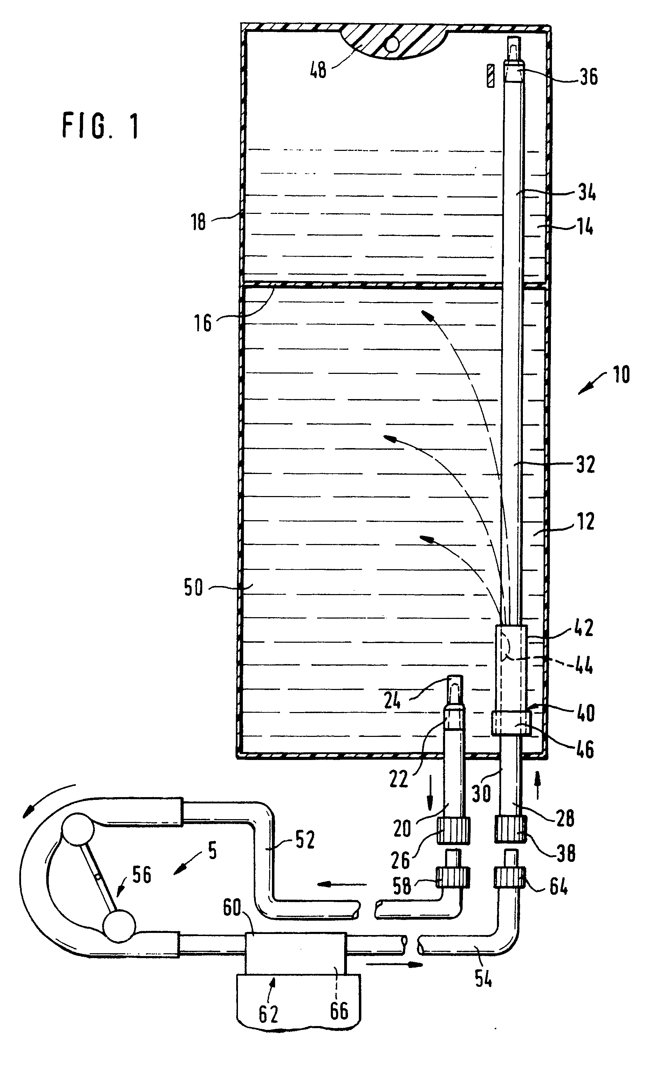

In Fig. 1 ist ein Beutelanordnung (10) gezeigt, die eine erste Beutelkammer (12) und eine zweite Beutelkammer (14) aufweist. Beide Beutelkammern (12) und (14) sind gemäß der in Fig. 1 gezeigten Ausführungsform durch eine gemeinsame Schweißlinie (16) voneinander getrennt und bilden demzufolge eine einzige Beuteleinheit, die von einem gemeinsamen Schweißrand (18) umgeben ist.

Die erste Beutelkammer (12) weist eine Auslaßleitung (20) auf, die gemäß der in Fig. 1 gezeigten Ausführungsform in den Schweißrand (18) eingeschweißt ist und durch diesen Schweißrand hindurch in der ersten Beutelkammer (12) mündet, diese also mit der Umgebung verbindet.

Im unbenutzten Zustand weist die Auslaßleitung (20) auf der Innenseite der ersten Beutelkammer (12) ein Abbrechteil (22) auf, dessen Ende (24) in an sich bekannter Weise abgebrochen werden kann, so daß hierdurch eine Strömungsverbindung zwischen der ersten Beutelkammer (12) und der Umgebung geschaffen wird.

Des weiteren weist das außerhalb der ersten Beutelkammer (12) befindliche Ende der Auslaßleitung (20) ein Konnektorstück (26) auf, das im unbenutzten Zustand mit einer Kappe steril verschlossen ist.

Die zweite Beutelkammer (14) weist demgegenüber eine Einlaßleitung (28) auf, die gemäß der in Fig. 1 gezeigten Ausführungsform sich durch die erste Beutelkammer (12) hindurch in die zweite Beutelkammer (14) erstreckt, so daß die Einlaßleitung aus folgenden Teilstücken besteht:
Einem außerhalb der Beutelanordnung (10) befindlichen Einlaßstück (30), einem in der ersten Beutelkammer (12) befindlichen Durchführungsstück (32) und einem in die zweite Beutelkammer (14) führenden Endstück (34), dessen Ende mit einem zweiten Abbrechteil (36) verbunden ist, das wie das vorstehend beschriebene Abbrechteil (22) gehandhabt wird.

Die Einlaßleitung (28) ist dabei jeweils mit dem umlaufenden Schweißrand (18) und mit der trennenden Schweißlinie (16) verschweißt, so daß zwischen den beiden Beutelkammern (12) und (14) und zur Umgebung hin kein Flüssigkeitsaustausch möglich ist.

Schließlich ist das Ende des Einlaßstücks (30) mit einem zweiten Konnektorstück (38) verbunden, das in seiner Funktion dem Konnektorstück (26) entspricht.

Gemäß der in Fig. 1 gezeigten Ausführungsform der Beutelanordnung (10) ist die Einlaßleitung im Bereich der ersten Beutelkammer (12), d.h. das Durchführungsstück (32), mit einem auf einen vorbestimmten Überdruck ansprechenden Ventil (40) versehen, das gemäß in der in Fig. 1 gezeigten Ausführungsform als elastisches Schlauchstück (42) ausgebildet ist, das elastisch dichtend am Durchführungsstück (32) anliegt und eine im Durchführungsstück (32) befindliche Öffnung (44) abdeckt. Vorteilhafterweise ist das Schlauchstück (42) mit Hilfe eines Fixierungsteils (46) am Durchführungsstück (32) fixiert und kann sich somit axial nicht verschieben.

Die Beutelanordnung (10) kann aus an sich bekannten Kunststoffmaterialien, beispielsweise PVC, Polyethylen, Polypropylen u.dgl. hergestellt sein, wobei diese Innenmaterialien üblicherweise außen mit einer Kaschierfolie, beispielsweise aus Polyamid, kaschiert sind.

Des weiteren kann - wie in Fig. 1 gezeigt - die Beutelanordnung (10) auf seiner Oberseite eine Aufhängeöse (48) aufweisen, mit der die Beutelanordnung (10) an einem nicht gezeigten Ständer aufgehängt sein kann.

Schließlich ist die erste Beutelkammer (12) mit einer Spülflüssigkeit (50) gefüllt, wobei das Aufnahmevermögen der ersten Kammer ca. $1^{-15}$1 beträgt. Demgegenüber beträgt das Aufnahmevermögen der zweiten Kammer (14) etwa 0,3 - 0,5 1.

Die Beutelanordnung (10) kann mit einer Perfusionseinrichtung (5) verbunden werden, die üblicherweise aus einem Blutschlauchsystem besteht, das einen arteriellen Zweig (52) und einen venösen Zweig (54) aufweist. In den arteriellen Zweig ist dabei eine peristaltische Pumpe (56) üblicherweise eingeschaltet. Das eine Ende des arteriellen Zweigs weist ein Konnektorstück (58) auf, das komplementär mit dem Konnektor (26) zusammenpaßt. Andererseits ist das andere Ende des arteriellen Zweigs mit der mit Blut beaufschlagbaren Kammer (60) des Dialysators (62) verbunden. Der Ausgang der mit Blut beaufschlagbaren Kammer (60) ist mit dem einen Ende des venösen Zweigs (54) verbunden, dessen anderes Ende mit einem weiteren Konnektorstück (64) verbunden ist, das zum Konnektorstück (38) komplementär ist.

Schließlich ist noch die mit (66) bezeichnete, von Dialysierflüssigkeit durchflossene Kammer des Dialysators (62) gezeigt, wobei die Flußrichtung der Dialysierflüssigkeit durch die Pfeilrichtung verdeutlicht ist.

Die in Fig. 1 gezeigte Anordnung wird folgendermaßen betrieben:
Zunächst wird das aus dem venösen und arteriellen Zweigen (52) und (54) bestehende Blutschlauchsystem mit dem Dialysator (62) verbunden und in die Pumpe (56) eingelegt. Anschließend werden die entsprechenden Konnektorstücke (26) und (58) bzw. (38) und (64) miteinander verbunden, so daß ein geschlossenes System entsteht. Schließlich werden die Abbrechteile (22) und (36) abgebrochen, so daß die erste Beutelkammer durch das Blutschlauchsystem und den Dialysator hindurch mit der zweiten Beutelkammer (14) in Strömungsverbindung gebracht wird. Danach wird die Pumpe (56) in Betrieb gesetzt, wobei zunächst die gesamte Luft aus dem Blutschlauchsystem und dem Dialysator verdrängt wird und sich in der zweiten Beutelkammer (14) sammelt. Anschließend füllt sich der arterielle Zweig (52), die Blutkammer (60) des Dialysators (62) und der venöse Zweig mit Spülflüssigkeit, die schließlich in der Einlaßleitung (28) hochsteigt und sich danach in der zweiten Beutelkammer (14) sammelt. Dieser Füllschritt dauert solange, bis sich das gesamte zur Verfügung stehende Innenvolumen der zweiten Beutelkammer (14) mit der Spülflüssigkeit nahezu gefüllt hat, wobei die in der zweiten Beutelkammer (14) angesammelte Luft von der Spülflüssigkeit komprimiert wird. Hierdurch steigt langsam der Innnendruck bis zu dem Wert, der dem Öffnungswert des Ventils (40) entspricht. Dieser liegt üblicherweise bei einem Überdruck von 0,1 - 0,3 bar. Sobald dieser Überdruck erreicht ist, öffnet das Ventil (40) und es fließt die Spülflüssigkeit wieder in rezirkulierender Weise in die erste Beutelkammer (12) zurück, während die verbrauchte, mit dem Konservierungsstoff beaufschlagte Spülflüssigkeit in der zweiten Kammer (14) zurückbleibt und aufgrund der Druckverhältnisse nicht zum Ventil (40) zurückfließt. Demzufolge wird also nach dem Öffnen des Ventils (40) im wesentlichen nur frische Spülflüssigkeit am Boden der ersten Kammer (12) abgezogen und stetig durch den Dialysator (62) geführt.

Nach einer bestimmten Perfusionsdauer wird die Pumpe (56) stillgesetzt und die Konnektorteile (26, 58) und (38, 64) voneinander gelöst, wobei die Konnektorstücke (58) und (64) anschließend mit Nadeln versehen werden, die danach in an sich bekannter Weise in den Patienten eingestochen werden.

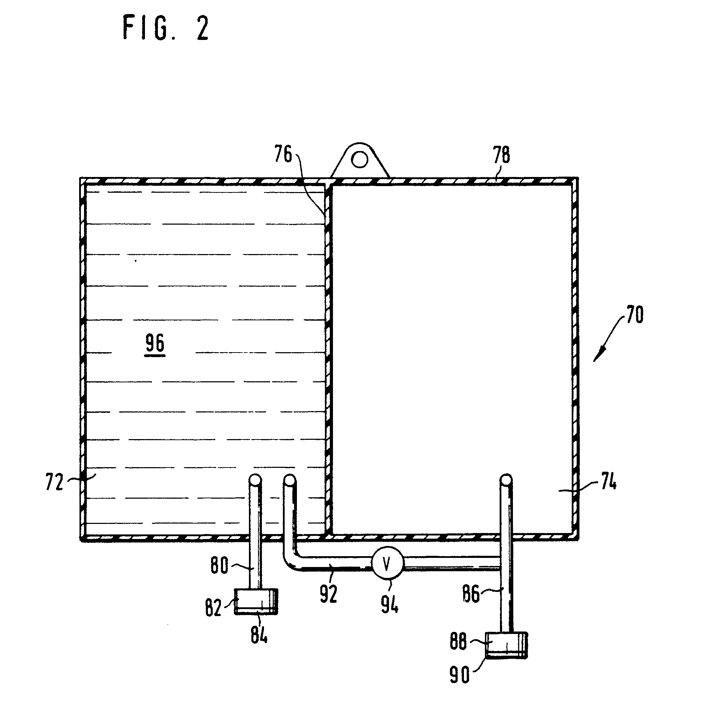

In Fig. 2 ist eine zweite Ausführungsform einer Beutelanordnung (70) gezeigt. Diese Beutelanordnung (70) weist eine erste Beutelkammer (72) und eine zweite Beutelkammer (74) auf, die ebenfalls eine gemeinsame Schweißlinie (76) besitzen. Gemäß einer weiteren Ausführungsform können jedoch die beiden Beutelkammern (72) und (74) auch in Form einzelner Beutel vorliegen, d.h. die gemeinsame Schweißlinie (76) ist entsprechend dieser Ausführungsform hier nicht mehr vorgesehen.

Des weiteren ist die Beutelanordnung (70) mit einem umlaufenden Schweißrand (78) versehen.

In die erste Beutelkammer (72) mündet eine Auslaßleitung (80), die an ihrem außerhalb der Beutelkammer liegenden Ende ein Konnektorstück (82) aufweist, das mit einer durchstechbaren Membran (84) versehen ist.

Die zweite Beutelkammer (74) weist eine Einlaßleitung (86) auf, die wie die Auslaßleitung (80) in den Beutelrand (78) eingeschweißt ist und diesen durchsetzt und in der zweiten Beutelkammer (74) mündet. Auch diese Einlaßleitung (86) weist an ihrem außerhalb der zweiten Beutelkammer (74) liegenden Ende ein Konnektorstück (88) auf, das ebenfalls an seinem Ende eine durchstechbare Membran (9) aufweist.

Von der Einlaßleitung (86) geht außerhalb der zweiten Beutelkammer (14) eine Überströmleitung (92) ab, deren Ende in der ersten Beutelkammer (72) mündet, wobei das Ende der Überströmleitung (92) in den Schweißrand (78) eingeschweißt ist und durch diesen hindurchführt. Des weiteren ist in die Überströmleitung (92) ein sich bei einem vorbestimmten Überdruck öffnendes Ventil (94) eingeschaltet, dessen Funktion der Funktion des Ventils (40) entspricht, das vorstehend beschrieben worden ist. Das Ventil kann als übliches, nach außen steril abgeschlossenes Rückschlagventil vorteilhafterweise ausgebildet sein, das sich aufgrund der Federwirkung bei einem bestimmten Überdruck, beispielsweise bei dem vorstehend geschilderten Überdruck, öffnet.

In der ersten Beutelkammer ist des weiteren eine Spüllösung (96) vorgesehen, die der vorstehend beschriebenen Spüllösung (50) entspricht.

Die beiden Konnektorstücke (82) und (88) können mit den Konnektorstücken (58) und (64) der arteriellen und venösen Blutleitung (52) und (54) verbunden werden, sofern diese Konnektorstücke mit den Nadelaufsätzen verbunden sind, damit die durchstechbaren Membranen (84) und (90) durchstochen werden können. Andererseits müssen jedoch diese durchstechbaren Membranen nicht vorgesehen sein, so daß die Konnektorstücke (82) und (90) ähnlich wie die Konnektorstücke (26) und (39) gemäß der in Fig. 1 beschriebenen ersten Ausführungsform komplementär zusammenpassen. Insofern können auch die Abbrechteile (22) und (36) bei den Auslaß- und Einlaßleitungen (80) und (86) vorgesehen sein.

Wesentlich an dieser zweiten Ausführungsform ist die separate Anordnung der beiden Leitungen (80) und (86), wobei von der Einlaßleitung (86) eine Überströmleitung (92) zur ersten Beutelkammer (72) führt, die bei einem bestimmten Überdruck durch das in sie eingeschaltete Überdruckventil (94) in Betrieb genommen wird. Die Arbeitsweise der in Fig. 2 gezeigten Ausführungsform entspricht der Arbeitsweise der in Fig. 1 gezeigten Ausführungsform, so daß auf deren Beschreibung Bezug genommen wird. So wird die Auslaßleitung (80) mit dem arteriellen Schlauchstück (52) verbunden, während die Einlaßleitung (86) mit dem venösen Schlauchstück (54) verbunden wird. Durch die Wirkung der Pumpe (56) leert sich die erste Beutelkammer (72), wobei zugleich die zweite Beutelkammer (74) bis zur Ausnutzung ihrer Füllkapazität aufgefüllt wird. Danach öffnet sich das vorteilhafterweise als Rückschlagventil ausgebildete Überdruckventil (94), wobei die umgepumpte Spülflüssigkeit über die Einlaßleitung (86) und die Überströmleitung (92) in die erste Beutelkammer (72) gepumpt wird und von dort wiederum in einem Rezirkulations-Kreislauf durch die Auslaßleitung (80) zum Dialysator (62) geführt wird.

Nach Beendigung der Perfusion werden die Konnektoren gelöst, wobei in üblicher Weise weiterverfahren wird.

## Ansprüche

1. Medizinische Beutelanordnung aus einem organischen Polymerisat zur Perfusion eines Geräts (62) zur Behandlung des menschlichen Körpers,
   mit einer ersten Beutelkammer (12; 72), die mit Spülflüssigkeit (50; 96) gefüllt ist und eine Auslaßleitung (20; 80) aufweist,
   mit einer zweiten Beutelkammer (14; 74), die eine Einlaßleitung (28; 86) aufweist,
   mit einer Verbindungsleitung (44; 92), die eine Strömungsverbindung zwischen der Einlaßleitung (28; 86) und der ersten Beutelkammer (12; 72) schafft, und
   mit einem Absperrorgan (40; 94), das in die Verbindungsleitung (44; 92) eingeschaltet ist,
   dadurch gekennzeichnet,
   daß das Absperrorgan als Ventil (40; 94) ausgebildet ist, das sich bei Erreichen eines vorbestimmten Druckes in der zweiten Beutelkammer, der durch in die zweite Beutelkammer (14; 74) geförderte verbrauchte Spülflüssigkeit (50; 96) und gegebenenfalls komprimierte Luft erzeugt wird, selbsttätig öffnet.

2. Medizinische Beutelanordnung nach Anspruch 1, dadurch gekennzeichnet, daß die Verbindungsleitung als Öffnung (44) ausgebildet ist, die in der Einlaßleitung (28) im Bereich der ersten Beutelkammer (12) angeordnet ist.

3. Medizinische Beutelanordnung nach Anspruch 2, dadurch gekennzeichnet, daß das Ventil (40) als elastisches Schlauchstück (42) ausgebildet ist, das die Einlaßleitung (28) elastisch umfaßt und die Öffnung (44) abdeckt.

4. Medizinische Beutelanordnung nach einem der Ansprüche 1-3, dadurch gekennzeichnet, daß das Ventil (40; 94) als Rückschlagventil ausgebildet ist.

5. Medizinische Beutelanordnung nach einem der Ansprüche 1-4, dadurch gekennzeichnet, daß die in den Beutelkammern (12, 14; 72; 74) vorliegenden Enden der Auslaßleitung (20; 80) und der Einlaßleitung (28; 86) jeweils ein Abbrechteil (22, 36) aufweisen.

6. Medizinische Beutelanordnung nach einem der Ansprüche 1-5, dadurch gekennzeichnet, daß die außerhalb der Beutelkammern (12, 14; 72, 74) befindlichen Enden der Auslaßleitung (20; 80) und der Einlaßleitung (28; 86) Konnektorstücke (26, 38; 82, 88) aufweisen, die in komplementärer Weise mit an einem Blutschlauchsystem (50) befindlichen Konnektorstücken (58, 64) verbindbar sind.

7. Medizinische Beutelanordnung nach Anspruch 1, dadurch gekennzeichnet, daß das Fassungsvermögen der ersten Beutelkammer (12; 72) etwa 1 bis 1,5 1 beträgt, und das Fassungsvermögen der zweiten Beutelkammer (14; 74) etwa 0,3-0,5 1 beträgt.

8. Medizinische Beutelanordnung nach Anspruch 1, dadurch gekennzeichnet, daß das Ventil (40; 94) bei einem Überdruck von 0,1 bis 0,3 bar öffnet.

## Claims

1. Medical bag arrangement of an organic polymer for perfusion in an apparatus (62) for treating the human body, comprising
a first bag chamber (12; 72) filled with a flushing liquid and having an outlet line (20; 80),
a second bag chamber (14; 74) having an inlet line (28; 86),
a connection line (44; 92) creating a flow connection between said inlet line (28; 86) and said first bag chamber (12; 72), and
a shut-off member (40; 94) connected into said connection line (44; 92),
characterized in
that said shut-off member is embodied as valve (40; 94) which opens automatically at a predetermined excess pressure in said second bag chamber, said excess pressure being generated by flushing liquid (50; 96) supplied to and used up in said second bag chamber (14; 74) and, if necessary, by compressed air.

2. Medical bag arrangement according to claim 1, characterized in that said connection line is embodied as opening (44) which is arranged in said inlet line (28) within the area of said first bag chamber (12).

3. Medical bag according to claim 2, characterized in that said valve (40) is embodied as flexible tube section (42) which resiliently engages round said inlet line (28) and covers said opening (44).

4. Medical bag arrangement according to any of claims 1-3, characterized in that said valve (40; 94) is embodied as return valve.

5. Medical bag arrangement according to any of claims 1-4, characterized in that the ends of said outlet line (20; 80) and of said inlet line (28; 86) lying in said bag chambers (12, 14; 72, 74) each have a break-off portion (22, 36).

6. Medical bag arrangement according to any of claims 1-5, characterized in that the ends of said outlet line (20; 80) and of said inlet line (28; 86) lying outside said bag chambers (12, 14; 72, 74) comprise connector pieces (26, 38; 82, 88) which are connectable in complementary manner to connector pieces (58, 64) disposed on a blood tubing system (50).

7. Medical bag arrangement according to claim 1, characterized in that the capacity of said first bag chamber (12; 72) is about 1-1.5 1 and that the capacity of said second bag chamber (14; 74) is about 0.3-0.5 1.

8. Medical bag arrangement according to claim 1, characterized in that said valve (40; 94) opens at an excess pressure of 0.1 to 0.3 bar.

## Revendications

1. Agencement de poche à usage médical en polymère organique pour la perfusion d'un appareil (62) pour le traitement du corps humain, comprenant une première chambre de poche (12; 72) qui est remplie d'un liquide de rinçage (50; 96) et qui présente un conduit de sortie (20; 80), une seconde chambre de poche (14; 74) qui présente un conduit d'entrée (28; 86), un conduit de raccordement (44; 92) qui établit une communication d'écoulement entre le conduit d'entrée (28; 86) et la première chambre de poche (12; 72), et un organe d'obturation (40; 94) qui est inséré dans le conduit de raccordement (44; 92), caractérisé en ce que l'organe d'obturation a la forme d'une valve (40; 94) qui s'ouvre automatiquement lorsque, dans la seconde chambre de poche, est atteinte une pression prédéterminée qui est produite par le liquide de rinçage (50; 96) souillé refoulé dans cette seconde chambre de poche (14; 74) ainsi que, le cas échéant, par de l'air comprimé.

2. Agencement de poche à usage médical suivant la revendication 1, caractérisé en ce que le conduit de raccordement a la forme d'une lumière (44) qui est ménagée dans le conduit d'entrée (28) dans la zone de la première chambre de poche (12).

3. Agencement de poche à usage médical suivant la revendication 2, caractérisé en ce que la valve (40) a la forme d'un bout de tuyau flexible élastique (42) qui entoure le conduit d'entrée (28) de manière élastique et qui recouvre la lumière (44).

4. Agencement de poche à usage médical suivant l'une quelconque des revendications 1 à 3, caractérisé en ce que la valve (40; 94) a la forme d'une valve de retenue.

5. Agencement de poche à usage médical suivant l'une quelconque des revendications 1 à 4, caractérisé en ce que les extrémités du conduit de sortie (20; 80) et du conduit d'entrée (28; 86) se trouvant à l'intérieur des chambres de poche (12, 14; 72, 74) présentent chacune une partie à briser (22, 36).

6. Agencement de poche à usage médical suivant l'une quelconque des revendications 1 à 5, caractérisé en ce que les extrémités du conduit de sortie (20; 80) et du conduit d'entrée (28; 86) se trouvant à l'extérieur des chambres de poche (12, 14; 72, 74) présentent des pièces de raccords (26, 38; 82, 88), qui peuvent être raccordées de manière complémentaire à des pièces de raccords (58, 64) faisant partie d'un système de circulation sanguine à tuyaux flexibles.

7. Agencement de poche à usage médical suivant la revendication 1, caractérisé en ce que la contenance de la première chambre de poche (12; 72) est de 1 à 1,5 1 et la contenance de la seconde chambre de poche (14; 74) est de 0,3 à 0,5 1.

8. Agencement de poche à usage médical suivant la revendication 1, caractérisé en ce que la valve (40; 94) s'ouvre sous une pression de 0,1 à 0,3 bar.

FIG. 1

# FIG. 2